(19) Europäisches Patentamt / European Patent Office / Office européen des brevets

(11) **EP 4 527 924 A2**

(12) **EUROPEAN PATENT APPLICATION**

(43) Date of publication:
26.03.2025 Bulletin 2025/13

(21) Application number: 24195540.0

(22) Date of filing: 21.08.2024

(51) International Patent Classification (IPC):
*C12N 15/10* (2006.01)  *C12N 15/64* (2006.01)
*C12N 15/67* (2006.01)  *C40B 40/08* (2006.01)

(52) Cooperative Patent Classification (CPC):
(C-Sets available)
**C12N 15/1065; C12N 15/64; C12N 15/67;**
**C40B 40/08** (Cont.)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 28.08.2023 JP 2023137652
19.06.2024 JP 2024098601

(71) Applicants:
• **Hitachi, Ltd.**
**Tokyo 100-8280 (JP)**
• **ARCALIS, Inc.**
**Minamisoma, Fukushima 975-0041 (JP)**

(72) Inventors:
• **KUMANO, Shun**
**Tokyo, 100-8280 (JP)**

• **NOJIMA, Akihiro**
**Tokyo, 100-8280 (JP)**
• **SUGIYAMA, Masuyuki**
**Tokyo, 100-8280 (JP)**
• **AKAHORI, Rena**
**Kashiwa-shi, 277-0871 (JP)**
• **KAWAKAMI, Shoko**
**Tokyo, 100-8280 (JP)**

(74) Representative: **Strehl & Partner mbB**
**Maximilianstrasse 54**
**80538 München (DE)**

Remarks:
The complete document including Reference
Table(s) and the Sequence Listing(s) can be
downloaded from the EPO website

(54) **METHOD FOR OPTIMIZING MRNA SEQUENCE USING PEPTIDE BARCODE**

(57) There is provided a method for optimizing a nucleic acid sequence, including the steps of: preparing a nucleic acid sequence that includes a candidate sequence containing a sequence of an untranslated region and a sequence encoding a target protein, and a sequence encoding a peptide barcode directly or indirectly linked to the target protein; expressing a protein from the nucleic acid sequence; separating the peptide barcode from the protein; analyzing the separated peptide barcode; and acquiring a relationship between expression of the target protein and the candidate sequence based on a result of the analysis, and selecting an optimal candidate sequence.

FIG. 1

**EP 4 527 924 A2**

(Cont. next page)

(52) Cooperative Patent Classification (CPC): (Cont.)

C-Sets
**C12N 15/1065, C12Q 2563/179**

**Description**

Cross-Reference to Related Applications

The present application claims the priority based on JP Patent Application No. 2023-137652 filed on August 28, 2023 and JP Patent Application No. 2024-098601 filed on June 19, 2024, all of which are incorporated by reference.

Technical Field

The present invention relates to a method and a kit for optimizing a nucleic acid sequence, e.g. the nucleic acid sequence of an mRNA drug.

Background Art

Nucleic acid drugs such as mRNA drugs have potential applications in infectious diseases and cancer treatments for many years. A nucleic acid (e.g. mRNA) encoding a protein to be expressed within the body is loaded on a carrier such as a lipid nanoparticle and introduced into the body. The nucleic acid (mRNA) taken up into the cell will express the encoded target protein according to a normal protein expression process. For example, when mRNA encoding a part of a virus is introduced as a vaccine to prevent infection, the expressed protein can be recognized by the immune system. Further, in the application as a cancer vaccine, mRNA encoding a specific antigen expressed in cancer cells, i.e. neoantigens, is introduced into the body. A treatment is performed such that the immune system is allowed to attack cancer cells in response of the recognition of the expressed neoantigens by the immune system. The nucleotide sequence of the nucleic acid (e.g. mRNA) can be designed to use the nucleic acid as a drug. Since the production method of the nucleic acid is simple, the sequence design, production, and shipment can be quickly performed. Because of these advantages, the application of nucleic acid (mRNA) drugs has been expected. During the COVID-19 pandemic, mRNA vaccines have been first put into practical use, demonstrating their performance.

Since the performance of nucleic acid drugs including mRNA depends on their nucleotide sequences, it is necessary to optimize the sequences for each target disease. For example, a nucleotide sequence of an mRNA drug generally includes a 5' untranslated region (5' UTR), an open reading frame (ORF) encoding a protein, and a 3' untranslated region (3' UTR). The 5' UTR is a site recognized by a ribosome and is involved in the control of expression. The ribosome translates into a protein based on the codons of the ORF. Expression efficiency varies depending on the patterns of the codons. The 3' UTR is a site related to the stability of mRNA. For example, the placement of Poly A containing contiguous adenines (A) in the 3' UTR has been known to improve the stability of mRNA, and the 3' UTR is also used in COVID-19 vaccines.

In the case of designing the nucleotide sequence of the mRNA drug, it is necessary to optimize the 5' UTR, ORF, and 3' UTR according to the purpose. Sequence optimization is important not only in the mRNA drug but also in the system involving the expression of proteins using DNA, and several studies have been conducted. A method for optimizing codons of ORFs is disclosed (Diez, M., et al. iCodon customizes gene expression based on the codon composition, Sci Rep 12 (1): 12126 (2022)). The software named iCodon makes it possible to improve the expression level by adjusting the codons. Further, a method for optimizing 5' UTR is disclosed (Sample, P.J., et al. Human 5' UTR design and variant effect prediction from a massively parallel translation assay, Nat Biotechnol 37 (7): 803-809 (2019)). A nucleotide sequence encoding a random amino acid sequence is located at a 5' UTR, and the 5' UTR to which a ribosome is likely to bind can be found by a polysome profiling technique.

With technical development of machine learning and deep learning, the accuracy of a sequence optimization calculation technique is improved. Meanwhile, it is not easy to acquire a large amount of molecular biological data for creating a learning model. When data indicating the relationship between the nucleotide sequence of the mRNA drug and the protein expression level is to be acquired by enzyme-linked Immunosorbent assay (ELISA), it is necessary to analyze one well for each sample whose nucleotide sequence has been changed. In respect of a method for analyzing myriad candidate substances in parallel, there are studies using peptide barcodes (JP 6781854 B; and Egloff, P., et al. Engineered peptide barcodes for in-depth analyses of binding protein libraries, Nat Methods, 16 (5): 421-428 (2019)). However, the techniques have been used for the purpose of screening substances that specifically bind to an antigen of interest, and are not used for designing the nucleotide sequence of the mRNA drug.

Summary of Invention

In the related art, in order to acquire the relationship between the nucleotide sequence and the protein expression level for optimizing nucleotide sequence of nucleic acid drugs such as mRNA drugs, it has been necessary to prepare one sample every time the nucleotide sequence is changed, prepare myriad samples, and analyze each of the samples. As the nucleotide sequence to be optimized increases in length, the candidate sequence increases exponentially. Thus, it has been substantially impossible to acquire data of the expression level.

In view of such a background, an object of the present invention is to provide a means and a method for acquiring data indicating a relationship between myriad candidate sequences in a nucleic acid (e.g. an mRNA drug) and an expression level of a protein in a high throughput manner, preferably by single measurement.

In the process of examining optimization of a nucleotide sequence of a nucleic acid (particularly an mRNA drug) for expressing a target protein, the present inventors have obtained knowledge that, upon the target protein expression from a candidate nucleic acid sequence, a target protein is expressed with linking to a peptide barcode capable of identifying the target protein, and the peptide barcode is analyzed, whereby a candidate nucleic acid sequence suitable for expression of the target protein can be selected, and have completed the present invention.

In one aspect, the present invention relates to a method for optimizing a nucleic acid sequence, including the steps of:

preparing a nucleic acid sequence that comprises a candidate sequence comprising a sequence of an untranslated region and a sequence encoding a target protein, and a sequence encoding a peptide barcode directly or indirectly linked to the target protein;
expressing a protein from the nucleic acid sequence;
separating the peptide barcode from the protein;
analyzing the separated peptide barcode; and
acquiring a relationship between expression of the target protein and the candidate sequence based on a result of the analysis, and selecting an optimal candidate sequence.

In another aspect, the present invention relates to a kit for use in optimizing a nucleic acid sequence, comprising a plurality of expression cassettes, the plurality of expression cassettes comprising:

an insertion site into which a candidate sequence comprising a sequence encoding a target protein and a sequence of an untranslated region are to be inserted; and
a sequence that encodes a peptide barcode comprising two or more amino acids,
in which each of the plurality of expression cassettes has a sequence encoding different peptide barcodes, and
when the expression cassettes are expressed, the target protein inserted into the insertion site is linked to the peptide barcode and expressed.

In other aspect, the present invention relates to a method for preparing a nucleic acid sequence that comprises a candidate sequence comprising a sequence of an untranslated region and a sequence encoding a target protein, and a sequence encoding a peptide barcode directly or indirectly linked to the target protein, comprising the steps of:

linking one or multiple types of sequences of a 5' UTR, one or multiple types of sequences encoding a target protein, one or multiple types of sequences of a 3' UTR, and sequences encoding multiple different types of peptide barcodes to plasmid vectors by DNA assembly method using homologous sequences to prepare plasmid vectors comprising the sequences in multiple combinations; and
amplifying the plasmid vectors,
wherein the DNA assembly method is performed under the condition that the number of types of the peptide barcodes is greater than the product of the number of types of the sequences of the 5' UTR, the number of types of the sequences encoding the target protein and the number of types of the sequences of the 3' UTR.

The present invention provides a method and a kit for optimizing a nucleic acid sequence as well as a method for preparing a nucleic acid sequence for use in the method. The use of the method and kit of the present invention enables the nucleotide sequence of a nucleic acid, e.g. an mRNA drug, to be easily optimized in a high throughput manner.

Brief Description of Drawings

FIG. 1 shows an outline of a first embodiment of the present invention in which sequence optimization of an mRNA drug is performed using peptide barcodes.
FIG. 2 shows images obtained by expressing mRNAs designed using two types of untranslated regions and corresponding peptide barcodes for expression of eGFP in cells, and then observing the fluorescence of the eGFP.
FIG. 3 shows a chromatogram obtained by expressing mRNAs designed using two types of untranslated regions and corresponding peptide barcodes for expression of eGFP in cells, and then analyzing the peptide barcodes on a liquid chromatography mass spectrometer (LCMS).

FIG. 4 shows an outline of an example for preparing a candidate nucleic acid sequence in sequence optimization of an mRNA drug.

FIG. 5 shows sequence configuration examples used in designing a candidate nucleic acid sequence.

FIG. 6 shows an outline of another example for preparing a candidate nucleic acid sequence in sequence optimization of an mRNA drug.

FIG. 7 shows an outline of the production of a plasmid vector for preparing a candidate nucleic acid sequence in sequence optimization of an mRNA drug.

FIG. 8 shows an outline of the preparation of a plasmid DNA containing a candidate sequence by DNA assembly method using homologous sequences.

FIG. 9 is a photograph showing how colonies were obtained on an agar medium plate in Example 2.

FIG. 10 shows a histogram of the lead length and frequency obtained by the nanopore sequencer in Example 2.

FIG. 11 is a graph showing the change in the overlap probability p of the peptide barcodes when changing m which is the number of types of the peptide barcodes.

Detailed Description of Embodiments

The present invention relates to a method and a kit for optimizing a nucleic acid sequence. According to the present invention, a sequence encoding a target protein and a sequence affecting expression (such as a sequence of an untranslated region) are linked to a sequence encoding an identifiable peptide barcode and expressed, and a peptide barcode portion in the expressed protein is analyzed, whereby a nucleic acid optimal for expression of the target protein can be selected based on the peptide barcode showing desired expression (high expression, long-term expression, etc.).

In one aspect, there is provided a method for optimizing a nucleic acid sequence, including the steps of:

preparing a nucleic acid sequence that includes a candidate sequence containing a sequence of an untranslated region and a sequence encoding a target protein, and a sequence encoding a peptide barcode directly or indirectly linked to the target protein;
expressing a protein from the nucleic acid sequence;
separating the peptide barcode from the protein;
analyzing the separated peptide barcode; and
acquiring a relationship between expression of the target protein and the candidate sequence based on a result of the analysis, and selecting an optimal candidate sequence.

The sequence optimization of a nucleic acid means that in order to express a target protein encoded by a nucleic acid, the sequence of the nucleic acid is optimized. The protein expression can vary depending on the sequence encoding the protein and the sequence of the untranslated region linked thereto. It may be preferable to obtain a nucleic acid having a sequence optimized to achieve the desired protein expression.

The nucleic acid may be RNA or DNA as long as it is a nucleic acid whose sequence is desired to be optimized for the protein expression. Preferably, the nucleic acid is RNA, e.g. an mRNA drug. The target protein encoded by the nucleic acid is not particularly limited as long as it is a protein whose expression is desired. In the case of a nucleic acid drug, e.g. an mRNA drug, the target protein may include, for example, proteins serving as immunogens of vaccines for infectious diseases (such as viruses, bacteria, and fungi), and proteins specifically expressed in cancer cells for cancer vaccines. Alternatively, the target protein may be a protein to be expressed in a large scale in a cell or in a cell-free expression system. The "nucleic acid sequence" used herein may be either DNA or RNA. For example, the term "nucleic acid sequence comprising/containing a sequence encoding a target protein" includes both a DNA sequence and an mRNA sequence obtained by reverse transcription from the DNA sequence.

According to the method of the present invention, a nucleic acid sequence including: a candidate sequence containing a sequence of an untranslated region and a sequence encoding a target protein; and a sequence encoding a peptide barcode directly or indirectly linked to the target protein may be prepared.

The untranslated region includes a 5' UTR and a 3' UTR. The untranslated region is a region that affects the protein expression. Accordingly, it is preferable to optimize the sequence of the untranslated region for desired protein expression. Only the 5' UTR or only the 3' UTR may be optimized, or both the 5' UTR and the 3' UTR may be optimized. As the candidate sequences of the 5' UTR and the 3' UTR, known untranslated region sequences or variants thereof may be used, or random sequences may be used.

According to the method of the present invention, identical nucleic acid sequences may be used as the sequence encoding the target protein. Alternatively, identical amino acids may be encoded by different codons due to genetic degeneracy. Thus, even when identical proteins are encoded, they have different nucleic acid sequences, and different sequences may cause different expression of the proteins. Therefore, the sequence encoding the target protein can also be subjected to optimization (codon optimization).

According to the method of the present invention, a candidate sequence including at least a sequence of an untranslated region and a sequence encoding a target protein may be linked to a sequence encoding a peptide barcode. The peptide barcode is a peptide composed of two or more amino acids, and each peptide barcode can be identified in the step of analyzing the peptide barcode. The peptide barcode may include, for example, a peptide consisting of 5 to 40 amino acids, preferably 10 to 30 amino acids. Preferably, the peptide barcode may have any length and composition which do not significantly affect the expression of the target protein.

In one embodiment, when the mass spectrometer is used in the step of analyzing the peptide barcode described later, a plurality of peptide barcodes used may include a sequence with high ionization efficiency. Here, the sequence with high ionization efficiency may preferably be longer than the length of other sequences in each of the peptide barcodes. For example, the plurality of peptide barcodes used may be designed such that at least a part of each of the barcodes includes an identical sequence, and the ionization efficiency of the sequence is high. In other words, the plurality of peptide barcodes may partially share common sequences with high ionization efficiency.

The untranslated region sequence, the sequence encoding the target protein, and the sequence encoding the peptide barcode can be prepared by a method known in the art.

In addition, the method of linking the candidate sequence to the sequence encoding the peptide barcode is well known in the art. For example, the sequence encoding the target protein and the sequence encoding the peptide barcode may be directly or indirectly linked such that the target protein and the peptide barcode are expressed as a fusion protein. The indirect linking can be performed via, for example, a sequence encoding an amino acid recognized by a protease, a spacer sequence, or the like. Examples of the amino acid recognized by the protease may include, but are not limited to, an amino acid DDDDK (SEQ ID NO: 1) recognized by an enterokinase, an amino acid recognized by trypsin, an amino acid recognized by thrombin, and an amino acid recognized by factor Xa. In one embodiment, the sequence encoding the peptide barcode may be linked to the sequence encoding the target protein via the sequence encoding the amino acid recognized by a protease.

A nucleic acid sequence including: a candidate sequence containing a sequence of an untranslated region and a sequence encoding a target protein; and a sequence encoding a peptide barcode directly or indirectly linked to the target protein may further include another sequence. In one embodiment, the sequence encoding the peptide barcode may be linked to a sequence encoding a purification tag. The purification tag may not be particularly limited as long as it is a tag commonly used in the art, and examples thereof may include a His tag, an HQ tag, and an HN tag (which can be purified by metal ions); a FLAG tag and the like (which can be purified by affinity chromatography); and an Myc tag and the like (which can be purified by an antibody). The purification tag may be used to easily purify and recover the peptide barcode to which the purification tag is linked or the peptide barcode and the target protein. The linkage between the sequence encoding the peptide barcode and the purification tag may be direct or indirect. For example, the sequence encoding the peptide barcode may be linked to the sequence encoding the purification tag via a sequence encoding an amino acid recognized by a protease. That is, in one embodiment, a sequence encoding an amino acid recognized by a protease may be present between the sequence encoding the peptide barcode and the sequence encoding the purification tag. The purification tag may be cleaved using the protease, and thus it is possible to avoid the influence of the purification tag in the peptide barcode analyzing step.

Alternatively or additionally, a nucleic acid sequence including: a candidate sequence containing a sequence of an untranslated region and a sequence encoding a target protein; and a sequence encoding a peptide barcode directly or indirectly linked to the target protein may be capped and/or poly A may be added to the nucleic acid sequence.

In one embodiment, in the preparing step of the method of the present invention, a nucleic acid sequence (including: a candidate sequence including a sequence of an untranslated region and a sequence encoding a target protein; and a sequence encoding a peptide barcode directly or indirectly linked to the target protein) may be prepared by amplifying the candidate sequence or the sequence encoding the target protein using a primer to which the sequence encoding the peptide barcode is added.

In one embodiment, in the preparing step of the method of the present invention, a nucleic acid sequence (including: a candidate sequence including a sequence of an untranslated region and a sequence encoding a target protein; and a sequence encoding a peptide barcode directly or indirectly linked to the target protein) may be prepared by amplifying the sequence encoding the target protein using a primer to which the sequence of the untranslated region is added.

In one embodiment, in the preparing step of the method of the present invention, a nucleic acid sequence (including: a candidate sequence including a sequence of an untranslated region and a sequence encoding a target protein; and a sequence encoding a peptide barcode directly or indirectly linked to the target protein) may be prepared by amplifying a plasmid vector containing the nucleic acid sequence.

In one embodiment, the preparing step of the method of the present invention may include:

preparing a plasmid vector containing a sequence of a 5' UTR that is the untranslated region, a plasmid vector containing the sequence encoding the target protein, and a plasmid vector containing the sequence encoding the peptide barcode;

preparing a plasmid vector containing the nucleic acid sequence from these vectors, the nucleic acid sequence including: a candidate sequence containing the sequence of the untranslated region and the sequence encoding the target protein, and the sequence encoding the peptide barcode; and

amplifying the plasmid vector containing the nucleic acid sequence to prepare the nucleic acid.

In addition to the plasmid vector described above, a plasmid vector containing an untranslated region: 3' UTR sequence may be further used to prepare the plasmid vector containing the nucleic acid sequence. The sequence of the 5' UTR and/or the sequence of the 3' UTR may include a specific candidate sequence or may be a random sequence.

In one embodiment, wherein the preparing step of the present invention comprises:

linking one or multiple types of sequences of an untranslated region, one or multiple types of sequences encoding a target protein, and sequences encoding multiple different types of peptide barcodes to plasmid vectors by DNA assembly method using homologous sequences to prepare plasmid vectors comprising the sequences in multiple combinations; and

amplifying the plasmid vectors to prepare the nucleic acid sequence that comprises the candidate sequence comprising the sequence of the untranslated region and the sequence encoding the target protein, and the sequence encoding the peptide barcode directly or indirectly linked to the target protein.

In order to prepare the plasmid vector containing the nucleic acid sequence, for example, a Gibson Assembly method using homologous sequences and a ligation method using blunt ends can be used. Both the methods are known in the art. Preferably, a plasmid vector containing the nucleic acid sequence may be prepared from each of the vectors or DNAs containing the sequences, the nucleic acid sequence including: a candidate sequence containing the sequence of the untranslated region and the sequence encoding the target protein; and the sequence encoding the peptide barcode, using the Gibson Assembly method. The Gibson Assembly method is a method used for linking a plurality of DNA fragments, and the method can link DNA fragments having a homologous sequence of a specific length (about 15 to 20 bases) at the end. Accordingly, in the case of using the Gibson Assembly method, the plasmid vector or DNA containing each sequence may be designed to contain a homologous sequence in the linking portion.

In one embodiment, the homologous sequence of the 5' UTR and the plasmid vector comprises a T7 promoter sequence. In one embodiment, the homologous sequence of the sequence of the 5' UTR and the sequence encoding the target protein comprises an initiation codon. In one embodiment, the homologous sequence of the sequence encoding the target protein and the sequence encoding the peptide barcode comprises a protease recognition sequence (a sequence encoding an amino acid recognized by a protease), e.g., a sequence encoding an amino acid recognized by an enterokinase. In one embodiment, the homologous sequence of the sequence encoding the peptide barcode and the sequence of the 3' UTR comprises a stop codon. In one embodiment, the homologous sequence of the sequence of the 3' UTR and the plasmid vector comprises a sequence of a portion of the plasmid vector (e.g., about 15 to 20 bases).

When multiple types of sequences are randomly linked to a plasmid vector, it is preferable to perform DNA assembly method under the condition that the number of types of the peptide barcodes is greater than the product of the number of types of the sequences of the 5' UTR, the number of types of the sequences encoding the target protein and the number of types of the sequences of the 3' UTR.

In a case where the nucleic acid whose sequence is to be optimized is RNA such as mRNA, a nucleic acid sequence can be prepared as RNA (mRNA) containing a candidate sequence by reverse transcription (e.g. in vitro transcription (IVT)) of a nucleic acid sequence (DNA) obtained by amplification.

In one embodiment, in the preparing step of the method of the present invention, a plurality of nucleic acid sequences may be prepared, the plurality of nucleic acid sequences including different untranslated region sequences and sequences encoding different peptide barcodes. The untranslated region sequences to be optimized may be each made to correspond to sequences encoding different peptide barcodes.

In one embodiment, in the preparing step of the method of the present invention, a plurality of nucleic acid sequences may be prepared, the plurality of nucleic acid sequences including different sequences encoding the target protein and sequences encoding different peptide barcodes. The sequences encoding the target protein to be optimized may be made to correspond to sequences encoding different peptide barcodes.

Subsequently, a protein may be expressed from the prepared nucleic acid sequence. In one embodiment, the expressing step of the method of the present invention may be performed in a cell. The cell may not be particularly limited, and may be a prokaryotic cell, for example, a bacterial cell (such as E. coli), or may be a eukaryotic cell, for example, a fungal cell (such as yeast), an insect cell, or a mammalian cell (such as human cell). Preferably, the cell may be a cell in which the target protein is expressed, and an optimal sequence for expression in the cell may be selected. For example, in the case of nucleic acid drugs for administration to humans, the expression in human cells may preferably be optimized.

In another embodiment, the expressing step of the method of the invention may be performed in a cell-free expression system. The cell-free expression system is also not particularly limited. An appropriate expression system can be used from among an expression system derived from E. coli, an expression system derived from wheat germ, an expression system derived from rabbit reticulocyte, and an expression system derived from insect cells, which are known in the art. A cell-free expression system mimicking a cell that is ultimately intended to express a target protein may preferably be used. For example, when a target protein is to be expressed in E. coli, a nucleic acid sequence can be easily and quickly optimized by using the expression system derived from E. coli.

Subsequently, a peptide barcode may be separated from an expressed protein. For separation of the peptide barcode, for example, when the expressed protein contains an amino acid recognized by a protease, the peptide barcode can be separated by using the protease.

The expressed protein and/or the separated peptide barcode may be purified at an appropriate stage, e.g. prior to analysis of the peptide barcode. The purifying step can be performed using a method used for purifying a protein, for example, a purification method using an antibody which binds to a target protein. In a case where a sequence encoding a purification tag is linked to the above-described nucleic acid sequence, the expressed protein and/or the separated peptide barcode can be easily purified using the purification tag.

Then, the separated peptide barcode may be analyzed. In one embodiment, the peptide barcode may be analyzed by a mass spectrometer. In another embodiment, the peptide barcode may be analyzed by a known protein analysis method (e.g. immunoassay such as ELISA or immunoblot). An apparatus and a method for analyzing peptide barcodes are well known in the art, and those skilled in the art can appropriately select the apparatus and method to be used depending on the composition and properties of the peptide barcode to be used.

In one embodiment, in the analyzing step of the method of the present invention, the ionic strength for each peptide barcode acquired by the mass spectrometer may be normalized by the ionization efficiency for each peptide barcode acquired in advance, and the abundance for each peptide barcode may be estimated from the normalized ionic strength.

In one embodiment, a mass-to-charge (m/z) peak list detected by the mass spectrometer may be created in advance based on the amino acid sequence length of each peptide barcode. In the analyzing step of the method of the present invention, the abundance of each peptide barcode may be estimated using the ionic strength of the m/z values in the m/z peak list.

In one embodiment, the method of the present invention further comprises:

sequencing the nucleic acid sequence contained in the plasmid vector and analyzing the abundance ratio for each sequence; and

normalizing the result of the analysis of the peptide barcodes with the abundance ratio, and acquiring relationship between expression of the target protein and the candidate sequence. After the nucleic acid sequences are prepared using the plasmid vector, the nucleic acid sequence(s) contained in the respective plasmid vectors may be sequenced, and the abundance ratio may be analyzed for each sequence. The abundance ratio can be used to normalize the abundance of the peptide barcodes to account for differences in the amount of nucleic acid sequence used.

According to the method of the present invention, a relationship between expression of the target protein and the candidate sequence may be acquired based on the result of the peptide barcode analysis, and an optimal candidate sequence may be selected. That is, since the peptide barcode corresponds to the candidate sequence, the relationship between expression of the target protein and the candidate sequence can be acquired by analyzing the peptide barcode. For example, it may be possible to acquire information on a candidate sequence corresponding to a target protein having a high or low expression level or a candidate sequence corresponding to a target protein having a long-term or short-term expression. Depending on the expression of the desired target protein, the optimal candidate sequence may be selected. In one embodiment, the candidate sequence having a high expression level of the target protein may be selected based on the result of the peptide barcode analysis. In one embodiment, the candidate sequence having a long-term expression of the target protein may be selected based on the result of the peptide barcode analysis.

The method of the present invention may further include a step of designing a primer based on an amino acid sequence of a peptide barcode corresponding to the selected candidate sequence, amplifying or reverse-transcribing at least a part of the nucleic acid sequence, sequencing the resulting sequence, and identifying the selected candidate sequence. In the case of using a random sequence as the candidate sequence (e.g. 5' UTR and/or 3' UTR), the sequence of the selected candidate sequence may not be known by analysis of the peptide barcode. Therefore, the sequence of the selected candidate sequence can be known by sequencing the sequence obtained by amplification or reverse transcription using a primer designed based on the amino acid sequence of the peptide barcode.

According to the method of the present invention, an identifiable peptide barcode is imparted to the expressed protein. Thus, even when a plurality of nucleic acids containing a candidate sequence for optimization is simultaneously expressed, the nucleic acids can be distinguished as expression products. Analyzing the separated peptide barcodes together makes it possible to simultaneously examine (screen) a plurality of candidate sequences. Therefore, according to the method of the present invention, the sequence of the nucleic acid can be easily optimized in a high throughput manner.

In another aspect, the present invention provides a kit for optimizing a nucleic acid sequence. Specifically, there is provided a kit, comprising a plurality of expression cassettes,
the plurality of expression cassettes including:

an insertion site into which a candidate sequence containing a sequence encoding a target protein and a sequence of an untranslated region are to be inserted; and
a sequence that encodes a peptide barcode containing two or more amino acids,
in which each of the plurality of expression cassettes has a sequence encoding different peptide barcodes, and
when the expression cassettes are expressed, the target protein inserted into the insertion site is linked to the peptide barcode and expressed.

An expression cassette can be in a form suitable for a cell or cell-free expression system for expressing a target protein, and can be, for example, in the form of a linear nucleic acid or a vector. The expression cassette may be either DNA or RNA. Preferably, the expression cassette may be DNA from the viewpoint of convenience of operation. When the sequence of RNA is optimized, RNA can be obtained by performing a reverse transcription reaction from an expression cassette DNA. Such an operation is well known in the art. The insertion site can be a site for inserting a sequence into the expression cassette, for example, a restriction site, a multiple cloning site, a homologous sequence, or the like.

The kit of the present invention may be used to contemplate that a nucleic acid whose sequence is desired to be optimized is linked to an insertion site of an expression cassette, a target protein is linked to a peptide barcode and expressed, and the target protein is easily expressed based on the peptide barcode.

The kit of the present invention may preferably be a kit for use in performing the method of the present invention described above (the method for optimizing a nucleic acid sequence). The method of the present invention can be easily and efficiently performed by using such a kit.

In other aspect, the present invention provides a method for preparing a nucleic acid sequence that comprises a candidate sequence comprising a sequence of an untranslated region and a sequence encoding a target protein, and a sequence encoding a peptide barcode directly or indirectly linked to the target protein, comprising the steps of:

linking one or multiple types of sequences of a 5' UTR, one or multiple types of sequences encoding a target protein, one or multiple types of sequences of a 3' UTR, and sequences encoding multiple different types of peptide barcodes to plasmid vectors by DNA assembly method using homologous sequences to prepare plasmid vectors comprising the sequences in multiple combinations; and
amplifying the plasmid vectors,
wherein the DNA assembly method is performed under the condition that the number of types of the peptide barcodes is greater than the product of the number of types of the sequences of the 5' UTR, the number of types of the sequences encoding the target protein and the number of types of the sequences of the 3' UTR.

The method for preparing a nucleic acid sequence according to the present invention can be used, for example, for preparing a nucleic acid sequence for use in the method for optimizing the sequence of a nucleic acid according to the present invention as described above. As the DNA assembling method using homologous sequences, for example, a Gibson Assembly method known in the art can be used. Preferably, Gibson Assembly method may be used to generate a plasmid vector comprising a nucleic acid sequence that comprises a candidate sequence comprising a sequence of an untranslated region and a sequence encoding a target protein, and a sequence encoding a peptide barcode from each of the DNAs or vectors containing the sequences. Gibson Assembly method is a method used for linking a plurality of DNA fragments, and DNA fragments having homologous sequences of a particular length (about 15 to 20 bases) at ends can be linked. Thus, when Gibson Assembly method is used, each of the vectors or DNA containing the sequences may be designed to contain homologous sequences in the linking moieties.

In one embodiment, the homologous sequence of the 5' UTR and the plasmid vector comprises a T7 promoter sequence.

In one embodiment, the homologous sequence of the sequence of the 5' UTR and the sequence encoding the target protein comprises an initiation codon.

In one embodiment, the homologous sequence of the sequence encoding the target protein and the sequence encoding the peptide barcode comprises a protease recognition sequence (a sequence encoding an amino acid recognized by a protease), e.g., a sequence encoding an amino acid recognized by an enterokinase.

In one embodiment, the homologous sequence of the sequence encoding the peptide barcode and the sequence of the 3' UTR comprises a stop codon.

In one embodiment, the homologous sequence of the sequence of the 3' UTR and the plasmid vector comprises a sequence of a portion of the plasmid vector (e.g., about 15 to 20 bases).

Hereinafter, modes for carrying out the present invention (referred to as "embodiments") will be described with reference to the attached drawings. Although the embodiments are specific examples according to the principles of the present invention, the embodiments are intended to promote understanding of the present invention, and should never be used to construe the technique of the present invention narrowly. Modified examples obtained by combining or replacing the following embodiments and known techniques are also included in the scope of the present invention. In all the drawings for describing the embodiments, components having the same function are denoted by the same reference signs, and the repeated description thereof will be omitted.

[First Embodiment]

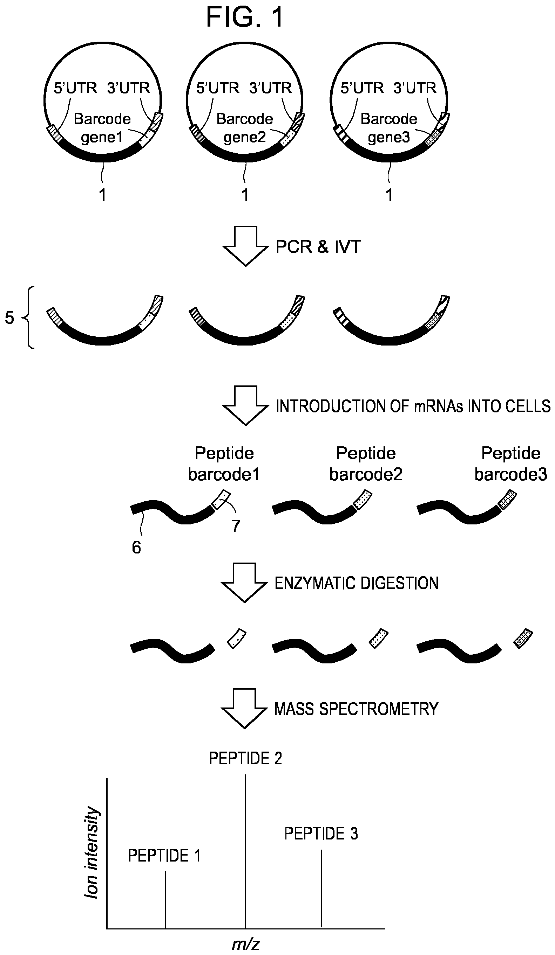

A first embodiment of the present invention will be described with reference to FIG. 1. In the first embodiment, a sequence encoding a target protein, a sequence of an untranslated region, and a sequence encoding a peptide barcode may be amplified as DNA, then transcribed to prepare mRNA, the mRNA may be introduced into a cell to express a protein, and the sequence of the mRNA may be optimized for expression of the target protein based on analysis of the peptide barcode in the expressed protein.

In FIG. 1, as an example, three patterns of untranslated region sequences are prepared, and three types of plasmid DNAs are prepared for selecting an optimal sequence for mRNA drug. In the three plasmids, genes (target DNAs) 1 encoding the target protein to be expressed are identical. Each of the DNAs is designed such that a peptide barcode is fused to the C-terminal side of the target protein. Each peptide barcode is composed of about 10 to 30 amino acids and is designed to correspond to each of three patterns of untranslated regions (5' UTR and 3' UTR) (Barcode gene 1, Barcode gene 2, and Barcode gene 3). That is, the sequence of the peptide serves as a barcode, and the correspondence with the untranslated region can be understood based on the barcode. A gene encoding an amino acid sequence recognized by a protease is inserted between a gene encoding a peptide barcode and a gene encoding a target protein. For example, in the case of using an enterokinase as the protease, the enterokinase recognizes an amino acid sequence represented by DDDDK (SEQ ID NO: 1), and cleaves the protein after K. Therefore, DNA (mRNA) may be designed such that at least the DDDDK sequence is inserted between the target protein and the peptide barcode.

In order to synthesize mRNA, linear DNAs are amplified by a polymerase chain reaction (PCR) from the three types of plasmid DNAs in FIG. 1. The linear DNAs are configured to contain a promoter. The promoter is, for example, a T7 promoter. DNAs are transcribed from the prepared linear DNAs into mRNAs 5 by IVT. At the time of PCR and IVT, a step of capping mRNA or adding Poly A may be added. As a 3' UTR, Poly A may be contained in each of the plasmid DNAs.

The transcribed mRNAs 5 are introduced into cells. The mRNAs can be introduced into cells using a gene transfer reagent such as a lipid. The introduction may be performed using an electroporation method. The mRNAs introduced into the cells are translated according to a normal protein expression process, and proteins 6 are synthesized. The amino acid sequences of the proteins 6 to be synthesized may change depending on the sequences of the genes (target DNAs) 1 encoding the proteins 6. Meanwhile, even when untranslated region sequences such as 5' UTR and 3' UTR are different, the amino acid sequences of the proteins 6 to be synthesized do not change. Therefore, the amino acid sequences of the target proteins 6 themselves derived from the three types of plasmid DNAs are identical. Although the target proteins 6 themselves do not change, peptide barcodes 7 are linked to the C-terminal sides, and the amino acid sequences of the peptide barcodes 7 are different for each untranslated region.

After the proteins 6 are expressed in cells, the cells are collected, and the target proteins 6 are recovered. In the case of proteins expressed in the cytoplasm, the proteins can be collected as a supernatant by lysis of cells with a normal lysis buffer and centrifugation thereof. The collected protein solution may be purified by acetone precipitation or the like to exchange the solvent, and the peptide barcodes 7 may be separated using a protease. In the case of an enterokinase, the enterokinase recognizes the DDDDK sequence (SEQ ID NO: 1) and cleaves the peptide bond after K, and each of the peptide barcodes 7 linked to the latter part may be separated from each of the proteins 6. The peptide barcodes 7 are expressed in a state of being fused with the target proteins 6, and the abundance of the peptide barcodes 7 means the expression level of the target proteins 6. When the expression level of the target proteins 6 varies for each untranslated region candidate sequence, the difference can be evaluated as the abundance of the peptide barcodes 7.

In order to evaluate the abundance of the peptide barcodes 7, sample solutions are analyzed by a mass spectrometer. In the case of evaluating the abundance of myriad peptide barcodes, it is desirable to use a high-resolution mass spectrometer because the difference in molecular weight between peptide barcodes is small. Meanwhile, in the case of measuring three types of peptide barcodes as in the example of FIG. 1, a device with high quantitativity may be selected, although the resolution is not as high as that of the triple quadrupole mass spectrometer. Further, in a case where peptide barcode portions are separately synthesized and the collision-induced dissociation conditions are examined in advance, the ionic strength can be evaluated by multiple reaction monitoring (MRM). The amount of the peptide barcodes correlates with the ion intensity obtained by the mass spectrometer, and thus it is found that the sequence of the untranslated region corresponding to the peptide barcode having a higher ion intensity has higher expression of the target protein. For example, since such an untranslated region sequence is an efficient untranslated region sequence for enhancing the expression of the target protein, it can be selected as an optimal sequence for enhancing the expression of the target protein.

[Example 1]

In this example, a principle verification result of the first embodiment of the present invention will be described. An enhanced green fluorescent protein (eGFP) was used as a target protein. Two patterns of untranslated regions (UTRs) were tested. The UTR of ATP5PF was used as the first UTR (UTR1), and the UTR of α-globin was used as the second UTR (UTR2). The sequences of UTR1 and UTR2 are shown below:

UTR1

3' UTR

AGAGCGGAGGTGGTGGCGGCGGAGGCTTTGGCAGCTCGGGACTGA

GTGCAAGAATCAGC (SEQ ID NO: 2)

5' UTR

AGAAATAAAGTAAAATTAATCTGGTAATTTGTCACGGATTAGTTGTAC

AACTAGTTAGAAGTTTCAGAATAAACATGCATTTCATAACTGTCAAATGTTCT

TTTAATTCTGAGTCCAAATAAATTATTTGGTGATGTTGA (SEQ ID NO: 3)

UTR2

5' UTR

3' UTR

ACTCTTCTGGTCCCCACAGACTCAGAGAGAACCCACC (SEQ ID NO: 4)

GCTGGAGCCTCGGTAGCCGTTCCTCCTGCCCGCTGGGCCTCCCAACG

GGCCCTCCTCCCCTCCTTGCACCGGCCCTTCCTGGTCTTTGAATAAAGTCTGA

GTGGGCAGC (SEQ ID NO: 5)

A plasmid DNA was designed such that a peptide barcode corresponding to the UTR1 was FVGARLDYKDDDDK (SEQ ID NO: 6) and a peptide barcode corresponding to the UTR2 was WLFPVGDYKDDDDK (SEQ ID NO: 8).

DNA sequence of a portion containing the peptide barcode corresponding to the UTR1 (encoding 6 amino acids as spacer, an enterokinase recognition sequence DDDDK (underlined), and a peptide barcode):

GGCGGTAGTGACTACAAA<u>GATGACGACGATAAA</u>TTTGTCGGTGCCCG

TCTAGACTACAAGGATGATGATGACAAG (SEQ ID NO: 7)

DNA sequence of a portion containing the peptide barcode corresponding to the UTR2 (encoding 6 amino acids as spacer, an enterokinase recognition sequence DDDDK (underlined), and a peptide barcode):

GGAGGCAGTGACTACAAA<u>GATGACGACGATAAA</u>TGGCTGTTCCCTGT

CGGAGACTATAAGGATGATGATGATAAG (SEQ ID NO: 9)

Prepared was a plasmid DNA in which DNA with the T7 promoter, the 3' UTR, the eGFP gene, the barcode gene, and the 5' UTR bound in this order was introduced into the multiple cloning site. A DNA fragment containing the T7 promoter and the 3' UTR was amplified by a PCR method to form a linear DNA. The DNA was purified by a DNA purification column, and then mRNA was synthesized by IVT using T7 RNA polymerase. The mRNA was purified by the RNA purification column, and then a cap structure was added to the mRNA. Again, the mRNA was purified by the RNA purification column and Poly A was added to the mRNA. Thereafter, the mRNA was purified again by the RNA purification column.

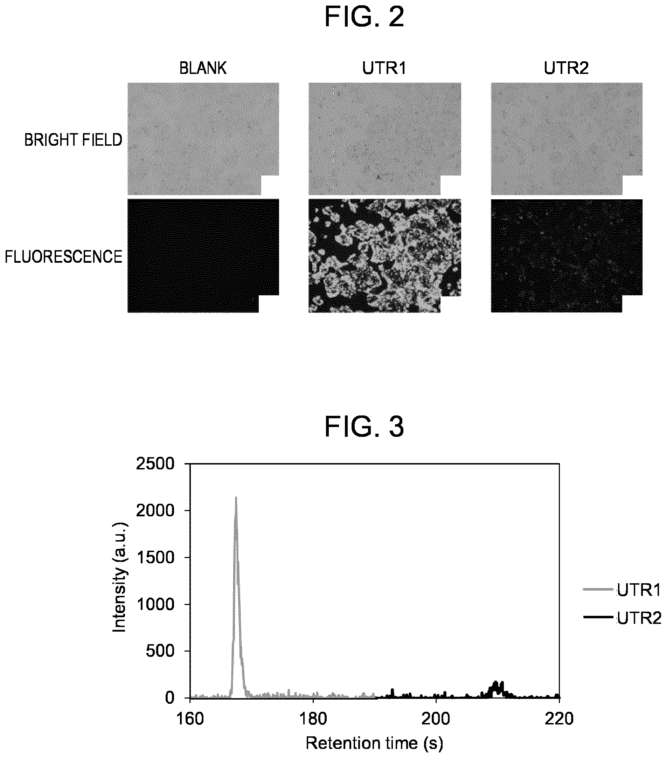

The purified mRNA was introduced into A431 cells (human epithelial cell-like) using an mRNA-introducing reagent. The eGFP green fluorescence was observed under a fluorescence microscope. Images obtained by observing the eGFP fluorescence are shown in FIG. 2. In a case where the eGFP is expressed using the UTR1 and the UTR2, it can be seen that the amino acid sequences of the eGFP moieties are identical, but there is a large difference in fluorescent brightness. Since the fluorescent brightness correlates with the eGFP expression level, the difference in brightness means a difference in expression level. It is found that the eGFP expression level in the case of using the UTR1 is increased as compared with the case of using the UTR2.

Further, the cells were collected and lysed in a lysis buffer before centrifugation. The supernatant was collected, and the protein was precipitated by an acetone precipitation method. The precipitate was treated with an enterokinase as the protease, thereby releasing the peptide barcodes. The peptide barcodes thus released were analyzed by measuring the peptide barcodes with LCMS. The results of the analysis are shown in FIG. 3. As separated by the LC column, peaks of the chromatogram are observed at different positions of the retention time, according to the peptide barcodes corresponding to the UTR1 and the UTR2. As is clear from the results, the peptide barcode released from the eGFP of the UTR1 has high intensity, and has a correlation with the fluorescence intensity shown in FIG. 2. Hence, the peptide barcode is measured with the mass spectrometer without observing the fluorescence intensity (expression of the target protein itself), and thus it is possible to determine which UTR has a higher expression level of eGFP, i.e. which UTR is effective for high expression.

In this example, two types of mRNAs were introduced into different cell samples, the difference in expression level could be grasped by the eGFP fluorescence (FIG. 2). In a case where the mRNAs are simultaneously introduced into one cell sample, even if the fluorescence is observed, it is not possible to determine whether the fluorescence is derived from the eGFP expressed from the mRNA of the UTR1 or the eGFP expressed from the mRNA of the UTR2. When the peptide barcode is used, such a problem does not occur. That is, a plurality of mRNAs is simultaneously introduced into one cell sample, the peptide barcodes are separated from the sample, and the peptide barcodes are measured with the mass spectrometer, and thus it is possible to examine which mRNA has a high protein expression level (FIG. 3). As the number of candidate sequences of mRNA increases, the method of the present invention utilizing a peptide barcode is more advantageous than a method of separately preparing cell samples and observing their expression.

[Modified Example of First Embodiment]

In FIGS. 1 to 3, mRNAs were introduced into cells, and the expression levels of the proteins were evaluated. Cultured cells may not necessarily be used for the confirmation of the expression. The target protein may be expressed by introducing mRNA into a cell-free expression system. The transition to automation in the use of the cell-free expression system is considered to be easier than that in the use of the cultured cells.

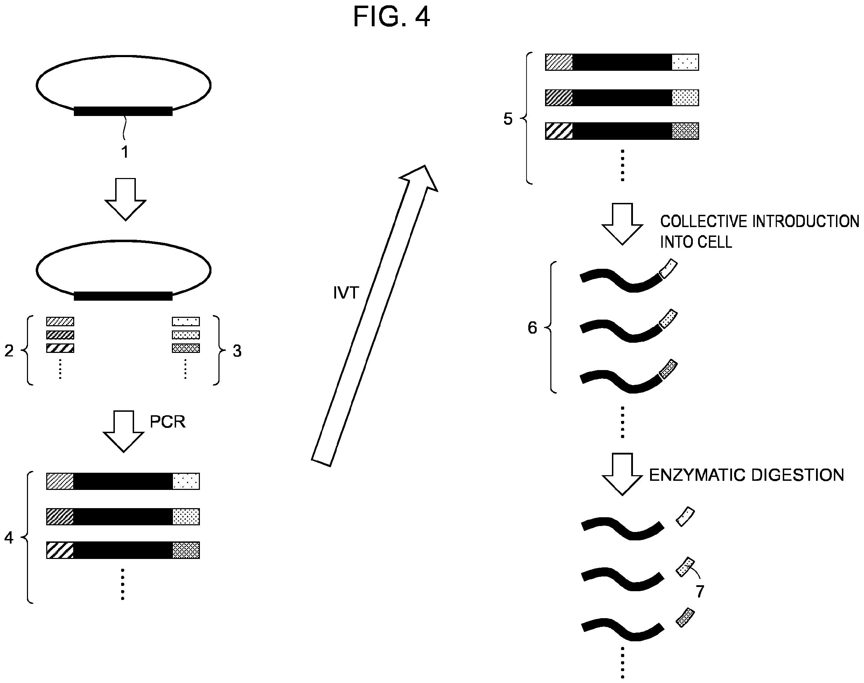

FIG. 4 shows an example of the process of preparing a plasmid DNA for optimization of 5' UTR. As an mRNA drug, a plasmid vector containing a gene (target DNA) 1 encoding a target protein desired to be expressed in the organism is prepared. The target DNA 1 is amplified from the plasmid vector by PCR. At that time, a 5' primer 2 to be used contains a 5' UTR candidate sequence, in addition to a portion complementary to a part of the target DNA. This is because the 5' UTR candidate sequence is added when the target DNA is amplified by PCR. The length of the 5' UTR candidate sequence is not limited, and the sequence may be randomly designed. The sequence may be combined with a known promoter sequence such as the T7 promoter. For example, a sequence in which the T7 promoter and a random nucleotide sequence of about 20 bases are connected may be used as the 5' UTR candidate sequence. During the PCR reaction, the plasmid vector is desirably mixed with a plurality of types of primers. In the case of using a 5' primer containing the sequence with the T7 promoter and the random nucleotide sequence of about 20 bases connected, a primer mix containing the 20 bases at random may be prepared and subjected to PCR reaction in one tube. As a result, a plurality of types of linear DNAs 4 including the candidate sequence is amplified.

Meanwhile, a 3' primer 3 contains a sequence complementary to the target DNA 1, a protease recognition sequence, a sequence encoding a peptide barcode, and a 3' UTR. The protease recognition sequence is a sequence recognized by a protease such as trypsin or enterokinase that cleaves a protein. For example, the enterokinase recognizes the amino acid sequence represented by DDDDK (SEQ ID NO: 1), and cleaves the protein after K (3' side). The peptide barcode 7 is a peptide in which two or more amino acids are linked, and is a substance used for linking the relationship between the candidate sequence and the expression level at the time of measurement with the mass spectrometer as described above. For example, Poly A is located at the 3' UTR. Similarly to the 5' primer 2, also in the 3' primer 3, it is preferable to prepare and use a primer mix obtained by mixing a plurality of types of primers including sequences encoding various types of peptide barcodes. Thus, the amplified PCR product will contain DNA encoding various peptide barcodes. When the 3' primer is designed, the sequence encoding the peptide barcode may be a random nucleotide sequence. When the nucleotide sequence is the random nucleotide sequence, the nucleotide sequence does not necessarily become a codon encoding an amino acid, and may become a nonsense codon. In this case, the length of the peptide barcode is shorter than originally expected. For example, in a case where the length of the peptide barcode is defined as 5 amino acids or more, when the length of the peptide barcode is 2 or 3 amino acids due to the presence of nonsense codon, the molecular weight is smaller than the initially defined molecular weight: 5 amino acids or more. Accordingly, it is possible to identify and delete a short peptide barcode from the data by providing a threshold with a defined molecular weight at the time of analysis by mass spectrometry.

The synthesis of the linear DNAs 4 is not necessarily performed by a single PCR. In the primer to be used, when the UTR sequence is much longer than the sequence portion complementary to the gene sequence of the target protein, stable amplification may not be achieved. Thus, for example, the 5' UTR and the portion of the target DNA 1 may be amplified by the first PCR. Subsequently, the PCR in which the peptide barcode side is added may be performed as the second PCR. After the first PCR, the linear DNAs 4 amplified may be purified by electrophoresis or the like, and then the next PCR may be performed.

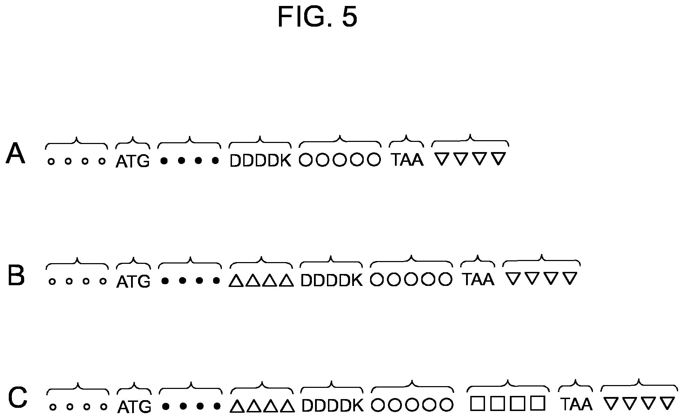

A of FIG. 5 shows an example of one of the linear DNAs 4 amplified by PCR. In the figure, circles, triangles, and squares mean positions of nucleotides, and the number thereof is not significant. A candidate sequence for 5' UTR (small open circles), such as a promoter or a random sequence, is arranged starting from the left, and then the start codon ATG is arranged. Subsequently, a target DNA 1 (small black circles) encoding a target protein is arranged, and a protease recognition sequence is arranged at the latter part. In FIG. 5, the DDDDK sequence (SEQ ID NO: 1) recognized by the enterokinase is inserted. The sequence encoding the peptide barcode 7 (open circles) is located at the back of the DDDDK sequence, and then the stop codon TAA is located. Finally, the 3' UTR (inverted triangles) is located. The sequence pattern is not limited to that shown in A of FIG. 5. In B of FIG. 5, a nucleotide sequence (triangles) encoding some amino acids is inserted as a spacer between the target DNA 1 (small black circles) and the protease recognition site. The function and expression may change depending on the C-terminal sequence of the protein. In the case of a protein having an important function at the C-terminus, the protein may cause steric hindrance with the amino acid sequence added at the C-terminus, leading to deterioration of the function. In such a case, it is effective to insert some amino acids (triangles) as a spacer between the target DNA 1 (small black circles) and the protease recognition site. In C of FIG. 5, a tag sequence (squares) is placed after the peptide barcode 7 (open circles). This is a tag sequence for purification such as a His tag or a FLAG tag. In a case where the amount of contaminants in samples during mass spectrometry described later is large and the measurement is hindered, it is desirable to perform the measurement after purifying the target protein using the tag.

As shown in FIG. 4, in a state where a plurality of types of PCR products is mixed, the DNAs 4 are transcribed into the mRNAs 5 by IVT. Thereafter, similarly to FIGS. 1 to 3, the peptide barcode 7 linked to the target protein 6 is measured with the mass spectrometer. Since myriad types of peptide barcodes 7 are contained in the sample solutions, a high-resolution mass spectrometer is desirably used. Peaks are classified based on slight differences in molecular weight resulted from a time-of-flight mass spectrometer or a Fourier transform mass spectrometer, and the peak intensity is acquired for each m/z ratio. The higher the concentrations in the sample solutions, the higher the peak intensity in the mass spectrum. Thus, a peak with high intensity may be extracted. It is necessary to extract a peak derived from the peptide barcode 7 among these peaks. The length of the peptide barcode or the range of the sequence length is fixed in the experiment. For example, the length is 10 amino acids, or the like. There are 20 types of amino acids. Assuming that 10 amino acids are randomly linked, it is possible to list the m/z ratios that can be detected by the mass spectrometer. The peak suitable for the list may be extracted. In a case where a plurality of peaks derived from the peptide barcode 7 and having high intensity is detected in a sample, the sample may be re-measured with a mass spectrometer for quantification to examine a more accurate quantification ratio. A triple quadrupole mass spectrometer is a representative example of the mass spectrometer for quantification. The high-resolution mass spectrometer can analyze a large amount of molecules at a time, but the quantitative accuracy is not high. Meanwhile, the mass spectrometer for quantification can analyze only a limited number of molecules in a single measurement, but has high quantitative accuracy. Therefore, it is reasonable to analyze some candidates picked up by the high-resolution mass spectrometer again with the mass spectrometer for quantification, in order to compare their abundance.

Not only the peptide barcode 7 but also various types of molecules are present in the sample solutions. Accordingly, depending on the state thereof, it may not be possible to determine which peak in the mass spectrum means the peptide barcode 7. In such a situation, the target protein 6 is desirably purified before treatment with a protease.

For example, an antibody that recognizes the target protein 6 can be used. The antibody is bound to the target protein 6 by binding the antibody to magnetic beads and mixing the magnetic beads with a solution containing the target protein 6. Only the magnetic beads are collected, and then the target protein 6 is separated from the antibody, allowing for purification. When a tag sequence is bound to the target protein 6 as shown in C of FIG. 5, an antibody that recognizes the tag can be used. In a case where the His tag is used, the tag binds to an immobilized metal and can be used for purification. The peptide barcode 7 may be separated from the purified protein, and the abundance of the peptide barcode 7 may be confirmed with the mass spectrometer as described above. In a case where the protease recognition sequence is inserted between the purification tag and the peptide barcode 7, the purification tag and the peptide barcode 7 can be separated by the protease. In the case of measurement with the mass spectrometer, a large molecular weight of the molecule to be measured may cause a decrease in ionization efficiency, and thus detection may not be possible. In such a case, desirably, the purification tag and the peptide barcode 7 may be separated to reduce the molecular weight, and only the peptide barcode 7 may be analyzed.

The peptide barcodes 7 contain different amino acids, and may differ in ionization efficiency. In a case where there is a difference of about 100 times in abundance, even when there is a difference in ionization efficiency for each of the peptide barcodes 7, it is possible to distinguish the difference in abundance by the ionic strength. However, when the difference is about several times, the difference may not be distinguished due to the difference in ionization efficiency. Therefore, a process of canceling the difference in ionization efficiency is desirably performed. One method is to produce a library including myriad peptide barcodes 7 and measure the library in advance with the high-resolution mass spectrometer. Thus, the ion intensity for each m/z of the detected peak can be used as a correction term of ionization efficiency. Normalizing the peak value of the mass spectrum by this correction term makes it possible to compare the abundances of the peptide barcodes 7 in a state of considering the difference in ionization efficiency. Another method is to equalize the ionization efficiencies of the peptide barcodes 7. The sequences of the peptide barcodes 7 do not need to be complete random sequences, and may partially share common sequences as long as the sequences can be separated based on an m/z value at the time of analysis with a mass spectrum or a spectrum using collision-induced dissociation. Attaching a peptide sequence with high ionization efficiency as a common sequence makes it possible to reduce the influence of portions other than the peptide sequence in each of the peptide barcodes 7. For example, the ionization efficiencies of the peptide barcodes 7 can be equalized by using a common sequence using molecules with high ionization efficiency, such as arginine and lysine. Further, in a case where the common sequence of the portion with high ionization efficiency is longer than the randomized sequence in each of the peptide barcodes 7, the effect of making the ionization efficiency constant may be high.

In a case where the purpose of sequence optimization is to maximize the expression level, the untranslated region sequence corresponding to the peptide barcode 7 with the highest abundance is selected. In a case where the purpose of sequence optimization is to achieve long-term expression, the untranslated region sequence corresponding to the peptide barcode 7 that has been detectable after long-term culture of mRNA-introduced cells and then analysis of the cells by the mass spectrometer is selected. Subsequently, the amino acid sequence of the peptide barcode 7 is calculated from the m/z value of the peptide barcode 7. In the identification of the amino acid sequence, if necessary, the identification accuracy is enhanced by adding cleavage information using collision-induced dissociation, electron transfer dissociation, or the like. Based on the determined amino acid sequence, a DNA sequence encoding the amino acid sequence is estimated, and a primer complementary to the DNA sequence is designed. The nucleotide sequences of the linear DNAs 4 or the mRNAs 5 containing the target DNA 1 are analyzed using the primer, thereby identifying the sequence of the 5' UTR corresponding to the selected peptide barcode.

In the synthesis example of plasmid DNA of FIG. 4, a method for optimizing a nucleic acid sequence using a plurality of patterns of 5' UTR sequences was shown as an example. The sequence to be optimized in the present invention is not limited to the 5' UTR. In the PCR step shown in FIG. 4, a plurality of patterns of 3' UTR sequence portions in the 3' primer 3 is prepared, similarly to the 5' primer 2, and thus the linear DNAs 4 having various 3' UTR sequences can be synthesized. Further, a plurality of patterns of linear DNAs 4 may be synthesized in which the codon is changed without changing the amino acid sequence of the ORF portion of the target protein. The subsequent steps are as described above. The abundance of the peptide barcode 7 is evaluated to search a candidate sequence effective in high expression and long-term expression.

The method of the present invention is a technique for characterizing a candidate sequence by matching a peptide barcode with a candidate sequence of a nucleic acid such as mRNA and evaluating the abundance of the peptide barcode. The sequence to be examined is the entire nucleic acid (e.g. mRNA) sequence, and is not limited to a certain portion.

[Second Embodiment]

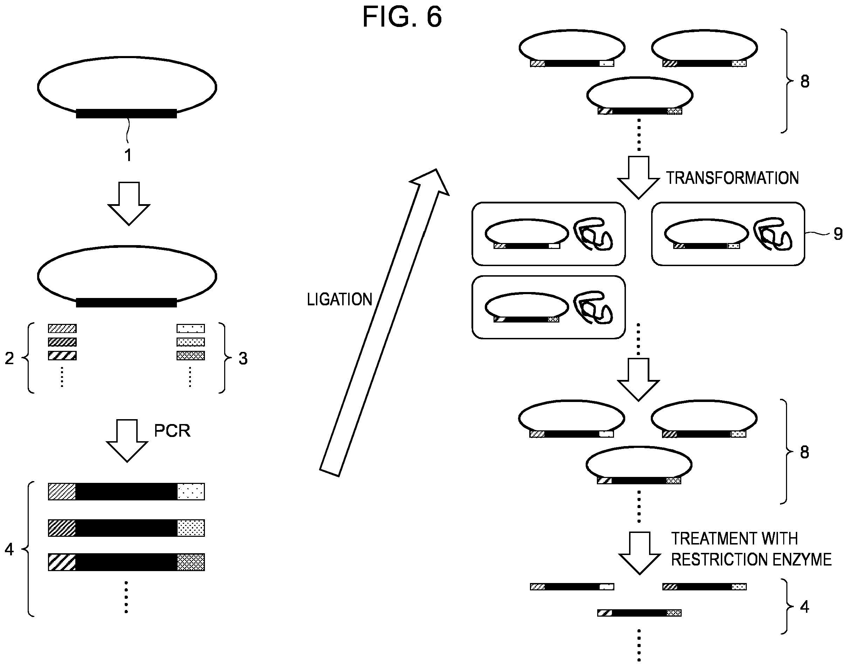

A second embodiment of the present invention will be described with reference to FIG. 6. In the second embodiment, similarly to the first embodiment, when DNA is amplified for preparing mRNA for the purpose of sequence optimization of an mRNA drug, a plasmid vector is used so as not to cause bias in DNA amplification efficiency.

FIG. 6 is an example of a plasmid DNA preparation process according to the second embodiment. The step of preparing the linear DNAs 4 containing the 5' UTR candidate sequence and the sequence encoding the peptide barcode 7 at both ends by PCR is the same as that in the first embodiment. However, the linear DNAs 4 do not necessarily contain the 3' UTR sequence. As the step after the synthesis of the linear DNAs 4, in the first embodiment, the mixture of the linear DNAs 4 was directly transcribed into the mRNAs 5 by IVT reaction. In a case where the amplification efficiency of PCR varies depending on the sequence, the amount of the mRNAs 5 per candidate sequence may not be uniform at the stage of transcription into the mRNAs 5. There is no problem as long as the variation in the level of the mRNAs 5 is sufficiently smaller than the variation in the expression level. However, when the variation in the level of the mRNAs 5 is larger than the variation in the expression level, it is unclear whether the analysis result corresponds to the influence of the expression level or the difference in the efficiency of the synthesis process of the mRNAs 5.

Accordingly, in the second embodiment, plasmid vectors 8 containing the linear DNAs 4 (genes encoding a target protein, a 5' UTR candidate sequence, a sequence encoding a peptide barcode) are prepared. A 3' UTR is included at the position where a linear DNA 4 is inserted into a plasmid vector 8, and when the linear DNA 4 is inserted, the 3' UTR is bound to the back of the termination codon of the linear DNA 4. Therefore, it is not necessary to bind the 3' UTR to the linear DNA in the synthesis of the linear DNAs 4, as in the first embodiment. Each of the produced plasmid vectors 8 is introduced into an E. coli 9 by transformation, followed by amplification. The efficiency of transformation is not high. Thus, there is a low possibility that, among the plasmid vectors 8 containing a large number of candidate sequences, a plural number of the plasmid vectors 8 containing an identical candidate sequence are incorporated into the E. coli. Hence, it can be assumed that the plasmid vectors containing the candidate sequences are incorporated, one by one, into the E. coli 9 according to one type, and the E. coli 9 in this state is amplified, and the plasmid vectors 8 are extracted. Thus, the variation in the amount for each candidate sequence is reduced as compared with the first embodiment. Further, a resistance gene of an antibiotic such as ampicillin resistance is inserted into each of the plasmid vectors 8, the resulting product is introduced into the E. coli 9 and cultured in a plating medium containing the antibiotic. Thus, the E. coli 9 having the plasmid vectors 8 forms colonies. The number of types of plasmid vectors 8 incorporated into the E. coli 9, i.e. the number of types of candidate sequences can be determined from the number of colonies formed. From the plasmid vectors 8 extracted from the E. coli 9, the linear DNAs 4 containing the candidate sequences are extracted with a restriction enzyme. The extracted linear DNAs 4 are transcribed into the mRNAs 5 by IVT reaction. The subsequent steps are as in the first embodiment.

Similarly to the first embodiment, in the present embodiment, the 5' UTR as an examination target for sequence optimization has been described as an example. However, the target is not limited to the 5' UTR. The primer during amplification in PCR may be designed to synthesize a plurality of 3' UTR candidate sequences.

[Third Embodiment]

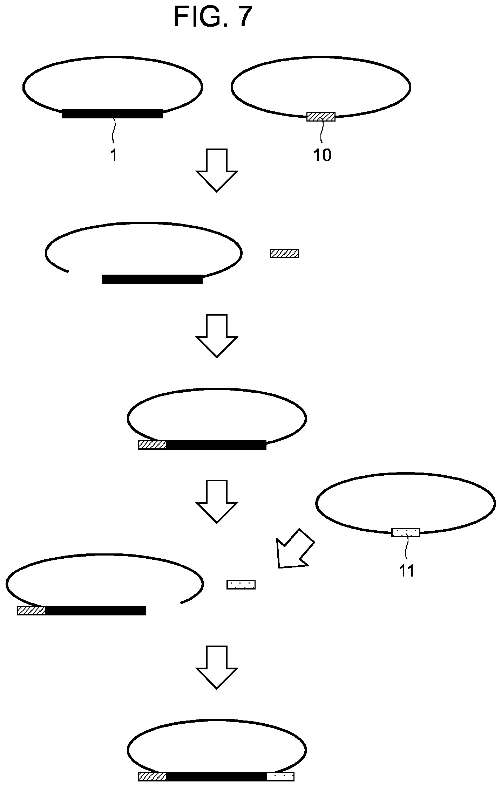

A third embodiment of the present invention will be described with reference to FIG. 7. FIG. 7 is a part of an analysis flow showing the third embodiment. In the present embodiment, similarly to the second embodiment, a 5' UTR candidate sequence 10, the target DNA 1, and a nucleotide sequence 11 encoding the peptide barcode are inserted into a plasmid vector, amplified in E. coli, and then transcribed into mRNA. FIG. 7 shows a flow for forming a plasmid vector. In advance, prepared are a plasmid vector into which the nucleotide sequence 11 encoding myriad types of peptide barcodes is inserted and a plasmid vector into which the 5' UTR candidate sequence 10 is inserted. For example, each of these plasmid vectors is produced by generating a linear DNA of the nucleotide sequence encoding the peptide barcode or the 5' UTR candidate sequence by DNA synthesis, and inserting the DNA into a plasmid vector by ligation using blunt ends or a method using homologous sequences, such as the Gibson Assembly system. As shown in FIG. 4, when the linear DNA is inserted into both ends of the target DNA 1, it is preferable to use a restriction enzyme that makes the ends to be blunt ends. After the plasmid vector in which the 5' UTR candidate sequence 10, the target DNA 1, and the nucleotide sequence 11 encoding the peptide barcode are inserted is generated, the subsequent process is the same as that of the second embodiment.

Similarly to the first embodiment, in the present embodiment, the 5' UTR as an examination target for sequence optimization has been described as an example. However, the target is not limited to the 5' UTR. Instead of the 5' UTR candidate sequence 10, the 3' UTR candidate sequence may be inserted into the plasmid vector by ligation using blunt ends or a method using homologous sequences, such as the Gibson Assembly system. Further, a plurality of 5' UTR and 3' UTR candidate sequences may be prepared and inserted into both ends of the target DNA 1.

[Fourth Embodiment]

In the fourth embodiment, a 5' UTR candidate sequence 10, a target DNA 1, a nucleotide sequence 11 encoding a peptide barcode, and a 3' UTR candidate sequence 12 are prepared as one or multiple types, respectively, and plasmid DNAs containing these sequences in random combinations are synthesized. As in the first to third embodiments, the multiple types of candidate sequences of the target DNA 1 differ in sequence, but the encoded amino acids and proteins are identical. DNA assembly method using homologous sequences such as Gibson Assembly may be used for the synthesis.

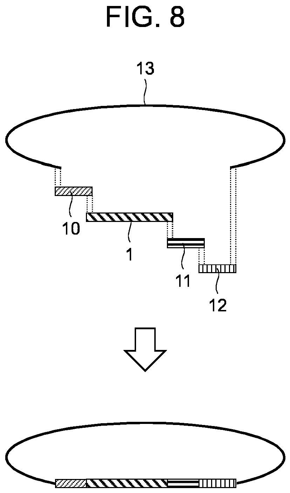

FIG. 8 shows methods for synthesizing a plasmid DNA according to the present embodiment. A plasmid DNA 13 (linearized with a restriction enzyme), 5' UTR candidate sequence 10 containing a T7 promoter, a target DNA 1, a nucleotide sequence 11 encoding a peptide barcode, and a 3' UTR candidate sequence 12 are prepared as linear DNA. In DNA assembly method, each sequence is linked by homologous sequences of about 15 bases. Thus, the leading portion of 5' UTR candidate sequence 10 and the binding portion of the linearized plasmid DNA 13, the back side of the 5' UTR candidate sequence 10 and the leading portion of the target DNA 1, the back side of the target DNA 1 and the leading portion of the nucleotide sequence 11 encoding the peptide barcode, the back side of the nucleotide sequence 11 encoding the peptide barcode and the leading portion of 3' UTR candidate sequence 12 as well as the back side of 3' UTR candidate sequence 12 and the binding portion of the linearized plasmid DNA 13 may be designed to each contain the same sequence (homologous sequence). Although the homologous sequence portion is fixed, the 5' UTR candidate sequence 10 containing T7 promoter, the target DNA 1, the nucleotide sequence 11 encoding the peptide barcode, and the 3' UTR candidate sequence may be prepared as one or more types, respectively, and mixed to link to the linearized plasmid DNA 13 as insert DNAs. Which sequences link is random, and multiple types of plasmid DNAs including a plurality of types of combinatorial sequences are synthesized. For example, when 5' UTR candidate sequences 10 containing T7 promoter, target DNAs 1, nucleotide sequences 11 encoding peptide barcodes, and 3' UTR candidate sequences 12 as three types, respectively, are mixed, $3^4$ types plasmid DNA are synthesized.

Insert DNAs are ligated to linearized plasmid DNA and then transformed into E. coli. Plasmid DNA contains a gene resistance to antibiotics such as kanamycin. When cultured in the presence of the antibiotic, only E. coli containing the plasmid DNA will survive. Colonies may be produced by culturing E. coli on agar plate medium. One colony can be thought of as a single plasmid DNA introduced into E. coli. Therefore, the number of colonies indicates the number of types of plasmid DNAs contained in the sample. Even if 1000 types of plasmid DNAs are theoretically synthesized from the combination DNA of inserts, if the number of colonies is 100, 100 types of the 1000 types of plasmid DNAs are included in the sample. After the E. coli is fully amplified, the plasmid DNA is extracted from the E. coli. At this point, the sequence of the plasmid DNA may be checked using a DNA sequencer such as a next-generation sequencer (NGS).

Subsequently, the portion required for mRNA synthesis may be amplified from the plasmid DNA by PCR. Alternatively, the plasmid DNA may be linearized using a restriction enzyme. Next, mRNA may be synthesized by IVT using T7 polymerase. When mRNA is synthesized, it is desirable to grasp the sequence and mRNA numbers of the sequences by the sequencer. mRNA count may vary from sequence to sequence, and the magnitude of mRNA count affects the level of expression of the protein after its introduction into the cell. If the number of mRNA per sequence is known prior to introduce into cells, the proportion can be used to correct the expression level.

After mRNA is introduced into the cells and the target protein is expressed, the cells are collected. After the protein is extracted from the cell, the peptide barcode may be released by a protease such as enterokinase. Peptide barcodes may be measured by mass spectrometry. If the sequence is checked in a state of mRNA, the sequence of the peptide barcode to be detected can be known in advance. By creating a m/z list based on this, the mass-spectrometry data can be efficiently analyzed. If necessary, collision-induced deviations may be used. Since the abundance of peptide barcode correlates with the amount of protein linked, the relationship between mRNA sequence and the amount of protein expression can be estimated based on the ion intensity of the peptide barcode. According to the present embodiment, a plurality of types of mRNA in which different peptide barcodes are linked to one type of mRNA sequence are generated. From the results of mass spectrometry, the ion intensities of the corresponding peptide barcodes can be averaged and compared to mitigate the effect of the characteristic differences of the peptide barcodes themselves on the results. The effects on the results mentioned here include the effect on the protein expression level caused by the linking of the peptide barcode and the difference in the ionization efficiency for each peptide barcode. In addition, as described above, the abundance of mRNA introduced into the cells may differ greatly from one combination pattern to another. In this case, by normalizing the ion intensity of each detected peptide barcode with the abundance of the combination pattern of mRNA, the effects of differing mRNA amounts can be mitigated.

According to the present embodiment, among combinations of 5' UTR candidate sequences 10, the target DNAs 1, and 3' UTR candidate sequences 12, the optimal one may be selected by using the peptide barcode. Therefore, specific peptide barcodes should correspond to combinations of the 5' UTR candidate sequence 10 containing each T7 promoter, the target DNA 1, and the 3' UTR candidate sequence 12 without overlap. For this purpose, in synthesizing the plasmid DNA, the number of types of the peptide barcodes needs to be sufficiently greater than the number of combinations, that is, the product of the number of types of: the 5' UTR candidate sequences 10 containing the respective T7 promoters, the target DNAs 1, the 3' UTR candidate sequences 12.

In DNA assembling method according to the present embodiment, homologous sequences are provided so as to form a margin at the leading portion and the back side of each of the combination sequences. This is also a limitation that reduces the degree of freedom of the sequence. For this reason, it is desirable to design the homologous sequence to be the minimum necessary. Further, in the present invention using the peptide barcode, it is preferable to minimize the decrease in the degree of freedom by incorporating the necessary sequence into the homologous sequence. For example, for the homologous sequence of the binding portion of the plasmid DNA and the 5' UTR candidate sequence 10 containing T7 promoter, it is desirable to incorporate T7 promoter sequence into the homologous sequence. T7 promoter is the sequence required to synthesize mRNA from DNA. Desirably, the homologous sequence of the binding portion of the target DNA 1 to the 5' UTR candidate sequence 10 containing T7 promoter contains ATG which is the initiation codon. This is necessary because it is translated from the initiation codon ATG. A DNA sequence encoding a protease-recognition sequence (an amino acid recognized by a protease) is preferably used as the homologous sequence of the binding portion between the target DNA 1 and the nucleotide sequence 11 encoding the peptide barcode. Protease recognition sequences are available to release the peptide barcode after protein expression. For example, an enterokinase recognizes DDDDK sequence (SEQ ID NO: 1) and cleaves it after K. Thus, linking the peptide barcode to the back of K results in the release of the peptide barcode from the protein upon treatment with enterokinase. In the homologous sequence of the junction between the nucleotide sequence 11 encoding the peptide barcode and the 3' UTR candidate sequence 12, it is desirable to include a stop codon. For example, TAA may be used. In order to design the gene such that the peptide barcode is linked to the C-terminus of the protein, it is preferred that the end of the sequence encoding the peptide barcode is a stop codon. Two codons may be overlapped to form a TAATAA or combined with other stop codons so that translation stops at the stop codon. For the homologous sequence of the binding portion between the 3' UTR candidate sequence 12 and the plasmid DNA 13, a sequence originally present in the plasmid DNA 13 (a sequence of a part of the plasmid DNA) may be incorporated into the 3' UTR candidate sequence 12 and used as a homologous sequence. In this instance, the degree of freedom in designing the candidate sequences of 3'UTR is not inhibited by homologous sequences. 3' UTR candidate sequences 12 may incorporate Poly A sequences. Poly A sequence contributes to the stability of mRNA. If a Poly A sequence is not introduced into 3' UTR candidate sequence 12, the primers can be designed such that Poly A is added during PCR in mRNA synthetic process. When Poly A sequence is included in 3'UTR candidate sequence 12, a linear DNA may be generated from the plasmid DNA using a restriction enzyme in addition to PCR method. A restriction enzyme site, e.g., *BspQI,* may be positioned behind Poly A. *BspQI* cleaves a sequence that is a distance from the restriction sequence. Therefore, the plasmid DNA can be linearized without an extra base being connected to Poly A.

[Example 2]

As an example of the fourth embodiment, an example is shown in which 5' UTR candidate sequences 10 containing three types of T7 promotors, three types of target DNAs 1 (eGFP gene), and nucleotide sequences 11 encoding approximately $2 \times 10^6$ types of protein barcodes are linked to a plasmid DNA 13 (3' UTR candidate sequence 12 has been previously inserted) using a DNA assembly method utilizing homologous sequences.

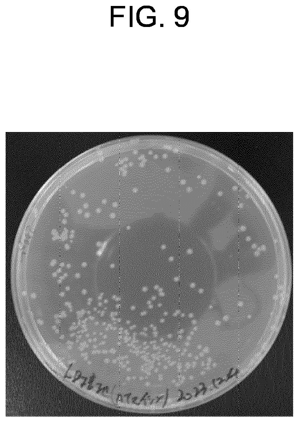

In this example, the combinations of the 5' UTR candidate sequences containing the three types of T7 promoters and the three types of target DNAs, i.e., the nine combinations, are evaluated. For the nine evaluations, about $2 \times 10^6$ types of peptide barcodes, which are sufficiently larger than the number of types, are designed to link to eGFP. During DNA assembly, the plasmid DNA is linearized in advance with a restrictive enzyme. Samples reacted with enzymes for DNA assembly were introduced into E. coli and then plated on agar plates containing kanamycin. FIG. 9 shows how a colony was obtained. More than 200 colonies have been obtained, each of which is assumed to contain one plasmid DNA. As described above, the present embodiment is intended to evaluate nine types of combinations. More than 200 colonies can be obtained, and it is expected that 9 types of combinations are included.

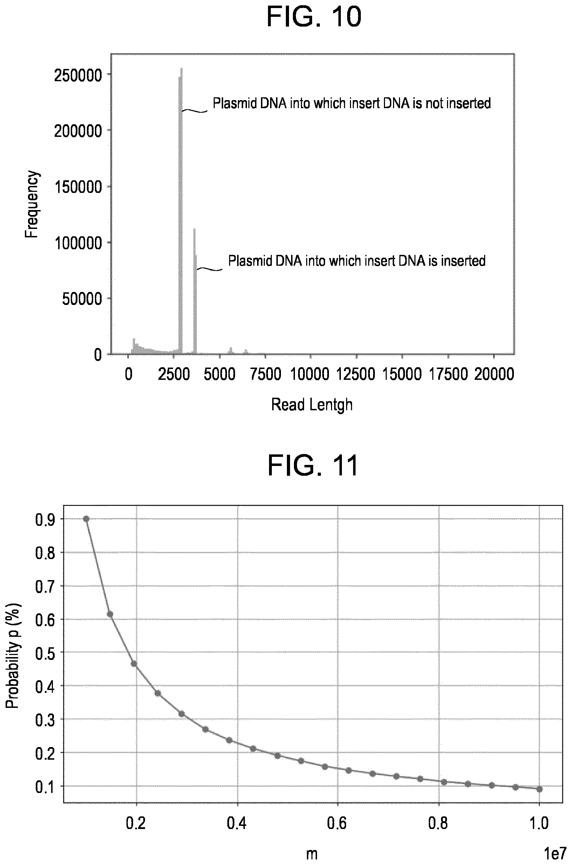

Approximately 200 colonies were collected and cultured in broth medium before plasmid DNA were collected from E. coli. Plasmid DNA were linearized with restriction enzymes and sequenced with a nanopore sequencer. A histogram of read lengths and frequencies detected by the nanopore sequencer is shown in FIG. 10. As shown in FIG. 10, two peaks appear, a plasmid DNA into which insert DNA is inserted and a plasmid DNA into which insert DNA is not inserted. When the insert DNA is inserted into the plasmid DNA by DNA assembly method, the insert DNA is not necessarily inserted into all the plasmid DNA, and the plasmid DNA may be self-ligated. Plasmid DNA that were not cleaved by restriction enzyme treatment prior to DNA assembly are also believed to remain. Such plasmid DNA have been introduced directly into E. coli and have also been detected during analyses with nanopore sequencers.

Table 1 below shows the combination patterns of insert DNA based on the nanopore sequencer analyses. 5' UTR candidate sequences 10 containing three types of T7 promoters are shown as U1, U2, and U3, and three types of target DNAs are shown as C1, C2, and C3. As shown in Table 1, all nine possible combinations are detected. In addition, it has been confirmed that a plurality of types of peptide barcodes are assigned to each combination pattern. It is preferable to normalize the expression level of the nucleic acid containing each combination according to the abundance ratio indicated in the detection number.

Table. 1

| Combination # | 5'UTR | Target DNA | Detection number | Number of types of barcodes |
|---|---|---|---|---|
| 1 | U1 | C1 | 4240 | 15 |
| 2 | U1 | C2 | 7148 | 19 |
| 3 | U1 | C3 | 4535 | 12 |
| 4 | U2 | C1 | 4860 | 18 |
| 5 | U2 | C2 | 9490 | 19 |
| 6 | U2 | C3 | 15955 | 25 |
| 7 | U3 | C1 | 4633 | 11 |
| 8 | U3 | C2 | 8663 | 11 |
| 9 | U3 | C3 | 13665 | 9 |

Table 2 provides a summary of the sequences of the peptide barcodes detected for each of the nine combinations. The combination numbers 1 to 9 in Table 2 correspond to Table 1. PB represents the respective peptide barcode (random sequence consisting of 6 amino acids) and PB of the same number represent the same peptide barcode.

Table. 2

| #1 | | #2 | | #3 | | #4 | | #5 | |
|---|---|---|---|---|---|---|---|---|---|
| Peptide barcodes | Detection Number | Peptide barcodes | Detection Number | Peptide barcodes | Detection Number | Peptide barcodes | Detection Number | Peptide barcodes | Detection Number |
| PB1 | 917 | PB16 | 700 | PB35 | 619 | PB47 | 919 | PB65 | 934 |
| PB2 | 469 | PB17 | 538 | PB36 | 396 | PB48 | 735 | PB66 | 620 |
| PB3 | 426 | PB18 | 432 | PB37 | 308 | PB49 | 289 | PB67 | 600 |
| PB4 | 251 | PB19 | 371 | PB38 | 293 | PB50 | 275 | PB68 | 559 |
| PB5 | 194 | PB20 | 323 | PB39 | 203 | PB51 | 189 | PB69 | 555 |
| PB6 | 171 | PB21 | 242 | PB40 | 106 | PB52 | 178 | PB70 | 412 |
| PB7 | 145 | PB22 | 234 | PB41 | 95 | PB53 | 169 | PB71 | 356 |
| PB8 | 124 | PB23 | 217 | PB42 | 89 | PB54 | 103 | PB72 | 328 |
| PB9 | 109 | PB24 | 183 | PB43 | 2 | PB55 | 101 | PB73 | 299 |
| PB10 | 78 | PB25 | 179 | PB44 | 1 | PB56 | 91 | PB74 | 270 |
| PB11 | 71 | PB26 | 172 | PB45 | 1 | PB57 | 75 | PB75 | 171 |
| PB12 | 46 | PB27 | 149 | PB46 | 1 | PB58 | 57 | PB76 | 165 |
| PB13 | 33 | PB28 | 106 | | | PB59 | 43 | PB77 | 147 |
| PB14 | 18 | PB29 | 95 | | | PB60 | 33 | PB78 | 96 |
| PB15 | 9 | PB30 | 75 | | | PB61 | 15 | PB79 | 57 |
| | | PB31 | 63 | | | PB62 | 7 | PB80 | 31 |
| | | PB32 | 62 | | | PB63 | 7 | PB81 | 30 |
| | | PB33 | 49 | | | PB64 | 1 | PB82 | 7 |
| | | PB34 | 34 | | | | | PB83 | 1 |

(continued)

| #6 | | #7 | | #8 | | #9 | |
|---|---|---|---|---|---|---|---|
| Peptide barcodes | Detection Number | Peptide barcodes | Detection Number | Peptide barcodes | Detection Number | Peptide barcodes | Detection Number |
| PB84 | 1383 | **PB109** | **798** | PB120 | 1759 | PB131 | 6000 |
| PB85 | 1176 | PB110 | 448 | PB121 | 897 | PB132 | 1097 |
| PB86 | 919 | PB111 | 378 | PB122 | 581 | PB133 | 522 |
| PB87 | 769 | PB112 | 355 | PB123 | 513 | PB134 | 270 |
| PB88 | 752 | PB113 | 285 | PB124 | 168 | PB135 | 269 |
| PB89 | 713 | PB114 | 264 | PB125 | 120 | PB136 | 186 |
| PB90 | 569 | PB115 | 185 | PB126 | 79 | PB137 | 177 |
| PB91 | 431 | PB116 | 134 | PB127 | 65 | PB138 | 136 |
| PB92 | 417 | PB117 | 109 | PB128 | 57 | **PB109** | **1** |
| PB93 | 346 | PB118 | 76 | PB129 | 45 | | |
| PB94 | 329 | PB119 | 50 | PB130 | 31 | | |
| PB95 | 322 | | | | | | |
| PB96 | 322 | | | | | | |
| PB97 | 265 | | | | | | |
| PB98 | 187 | | | | | | |
| PB99 | 166 | | | | | | |
| PB100 | 148 | | | | | | |
| PB101 | 132 | | | | | | |
| PB102 | 89 | | | | | | |
| PB103 | 52 | | | | | | |
| PB104 | 42 | | | | | | |
| PB105 | 1 | | | | | | |
| PB106 | 1 | | | | | | |
| PB107 | 1 | | | | | | |
| PB108 | 1 | | | | | | |

As shown in Table 2, sequences approximately specific for the peptide barcode sequences in each combination have been detected. However, in combination #7 and combination #9, there was an overlap in the peptide barcode sequence (PB109). In this example, peptide barcodes consisting of a variety of amino acids are randomly inserted into the plasmid DNA and a part thereof is introduced into E. coli to form a colony. This results in the occurrence of peptide barcode overlap with a certain probability. To reduce this rate of overlap, the number of sequences encoding the peptide barcode may be increased in performing DNA assembly method. In addition, if the duplicated peptide barcodes are not used for analysis in mass spectrometry, the overlap does not affect the object of the present invention (the expression level of the candidate nucleic acid sequence is analyzed by correlating the peptide barcodes). Therefore, even if several peptide barcodes overlap, they can be omitted from the analysis, which is not a question.

The probability p of overlapping peptide barcodes is considered. Assuming that the number of types of peptide barcodes is m, the combination pattern of plasmid DNA is n, and the number of types of peptide barcodes assigned to each combination pattern of plasmid DNA is k, p is 1 minus the probability that all peptide barcodes differ, and is expressed by the following equation.

$$p(\%) = (1 - \frac{m!}{(m - kn)! \cdot m^{kn}}) \cdot 100$$

For example, assuming that m = $2\times10^6$ types, n=9, and k is 15, p = 0.45% can be calculated. This calculation assumes that for all peptide barcode sequences, the likelihood of inserting into the plasmid DNA is constant. In DNA assembly method, there is a possibility that the synthesis probability differs for each sequence, the probability of introduction into E. coli, the growth rate of E. coli, and the like may vary depending on the sequence, which may be an error factor in the above calculation. FIG. 11 shows the change in the overlap probability p when the number of types m of the peptide barcode is changed. It can be seen that p decreases as the number m of types of peptide barcodes increases. However, it can be seen that the decrease in p is not so large even if it is increased from the current $2\times10^6$ types. Further, considering that there was one overlap even under the conditions of this example of p = 0.45%, if the experiment under the conditions of p<0.5%, it is considered that the object of the present invention can be achieved.

The present invention is not limited to the embodiments described above, and various modified examples are included. For example, the above-described embodiments have been described in detail in order to explain the present invention in an easy-to-understand manner, and are not necessarily limited to those having all the configurations described. Further, a part of the configuration of a certain embodiment can be replaced with the configuration of another embodiment, and the configuration of another embodiment can be added to the configuration of the certain embodiment. Besides, a part of the configuration of each embodiment can be added to the configuration of another embodiment, can be deleted, and can be replaced with the configuration of another embodiment.

Reference Signs List

1 gene encoding target protein (target DNA)
2 5' primer containing 5' UTR candidate sequence
3 3' primer containing nucleotide sequence encoding peptide barcode
4 linear DNA
5 mRNA
6 target protein
7 peptide barcode
8 plasmid vector containing gene encoding target protein, 5' UTR candidate sequence, and sequence encoding peptide barcode
9 E. coli
10 5' UTR candidate sequence
11 nucleotide sequence encoding peptide barcode
12 3' UTR candidate sequence
13 linearized plasmid DNA

**Claims**

1. A method for optimizing a nucleic acid sequence, comprising the steps of:

   preparing a nucleic acid sequence that comprises a candidate sequence comprising a sequence of an untranslated region and a sequence encoding a target protein, and a sequence encoding a peptide barcode directly or indirectly linked to the target protein;
   expressing a protein from the nucleic acid sequence;
   separating the peptide barcode from the protein;
   analyzing the separated peptide barcode; and
   acquiring a relationship between expression of the target protein and the candidate sequence based on a result of the analysis, and selecting an optimal candidate sequence.

2. The method according to claim 1, wherein in the preparing step, a plurality of nucleic acid sequences is prepared, the plurality of nucleic acid sequences comprising:

   different untranslated region sequences and sequences encoding different peptide barcodes, and/or
   different sequences encoding the target protein and sequences encoding different peptide barcodes.

3. The method according to claim 1 or 2, wherein the nucleic acid is an mRNA drug.

4. The method according to any preceding claim, wherein the expressing step is performed in a cell or in a cell-free expression system.

5. The method according to any preceding claim, wherein

in the preparing step, the nucleic acid sequence is prepared by amplifying the candidate sequence or the sequence encoding the target protein using a primer to which the sequence encoding the peptide barcode is added, or
in the preparing step, the nucleic acid sequence is prepared by amplifying the sequence encoding the target protein using a primer to which the sequence of the untranslated region is added, or
in the preparing step, the nucleic acid sequence is prepared by amplifying a plasmid vector containing the nucleic acid sequence, or
the preparing step comprises:

preparing a plasmid vector comprising a sequence of a 5' untranslated region (5' UTR) that is the untranslated region, a plasmid vector comprising the sequence encoding the target protein, and a plasmid vector comprising the sequence encoding the peptide barcode;
preparing a plasmid vector comprising the nucleic acid sequence from these vectors, the nucleic acid sequence comprising: a candidate sequence comprising the sequence of the untranslated region and the sequence encoding the target protein, and the sequence encoding the peptide barcode; and
amplifying the plasmid vector comprising the nucleic acid sequence to prepare the nucleic acid sequence.

6. The method according to any preceding claim, wherein

the sequence encoding the peptide barcode is linked to a sequence encoding a purification tag, and preferably a sequence encoding an amino acid recognized by a protease is present between the sequence encoding the peptide barcode and the sequence encoding the purification tag.

7. The method according to any preceding claim, wherein the preparing step comprises:

linking one or multiple types of sequences of an untranslated region, one or multiple types of sequences encoding a target protein, and sequences encoding multiple different types of peptide barcodes to plasmid vectors by DNA assembly method using homologous sequences to prepare plasmid vectors comprising the sequences in multiple combinations; and
amplifying the plasmid vectors to prepare the nucleic acid sequence that comprises the candidate sequence comprising the sequence of the untranslated region and the sequence encoding the target protein, and the sequence encoding the peptide barcode directly or indirectly linked to the target protein.

8. The method according to claim 7, further comprising:

sequencing the nucleic acid sequence contained in the plasmid vector and analyzing the abundance ratio for each sequence; and
normalizing the result of the analysis of the peptide barcodes with the abundance ratio, and acquiring relationship between expression of the target protein and the candidate sequence.

9. The method according to any preceding claim, wherein the sequence encoding the peptide barcode is linked to the sequence encoding the target protein via a sequence encoding an amino acid recognized by a protease.

10. The method according to any preceding claim, wherein the peptide barcode is analyzed by a mass spectrometer.

11. The method according to any preceding claim, wherein

the candidate sequence having a high expression level of the target protein is selected based on the result of the analysis, or
the candidate sequence having a long-term expression of the target protein is selected based on the result of the analysis.

12. The method according to any preceding claim, further comprising a step of designing a primer based on an amino acid

EP 4 527 924 A2

sequence of a peptide barcode corresponding to the selected candidate sequence, amplifying or reverse-transcribing at least a part of the nucleic acid sequence, sequencing the resulting sequence, and identifying the selected candidate sequence.

13. A kit for use in optimizing a nucleic acid sequence preferably in performing the method of any of claims 1 to 12, the kit comprising a plurality of expression cassettes, the plurality of expression cassettes comprising:

an insertion site into which a candidate sequence containing a sequence encoding a target protein and a sequence of an untranslated region are to be inserted; and
a sequence that encodes a peptide barcode comprising two or more amino acids,
wherein each of the plurality of expression cassettes has a sequence encoding different peptide barcodes, and when the expression cassettes are expressed, the target protein inserted into the insertion site is linked to the peptide barcode and expressed.

14. A method for preparing a nucleic acid sequence that comprises a candidate sequence comprising a sequence of an untranslated region and a sequence encoding a target protein, and a sequence encoding a peptide barcode directly or indirectly linked to the target protein, comprising the steps of:

linking one or multiple types of sequences of a 5' untranslated region (5' UTR), one or multiple types of sequences encoding a target protein, one or multiple types of sequences of a 3' untranslated region (3' UTR), and sequences encoding multiple different types of peptide barcodes to plasmid vectors by DNA assembly method using homologous sequences to prepare plasmid vectors comprising the sequences in multiple combinations; and amplifying the plasmid vectors,
wherein the DNA assembly method is performed under the condition that the number of types of the peptide barcodes is greater than the product of the number of types of the sequences of the 5' UTR, the number of types of the sequences encoding the target protein and the number of types of the sequences of the 3' UTR.

15. The method according to claim 14, wherein

the homologous sequence of the 5' UTR and the plasmid vector comprises a T7 promoter sequence, and/or the homologous sequence of the sequence of the 5' UTR and the sequence encoding the target protein comprises an initiation codon, and/or
the homologous sequence of the sequence encoding the target protein and the sequence encoding the peptide barcode comprises a sequence encoding an amino acid recognized by a protease, and/or
the homologous sequence of the sequence encoding the peptide barcode and the sequence of the 3' UTR comprises a stop codon, and/or
the homologous sequence of the sequence of the 3' UTR and the plasmid vector comprises a sequence of a portion of the plasmid vector.

22

# FIG. 1

## FIG. 2

|  | BLANK | UTR1 | UTR2 |
| --- | --- | --- | --- |
| BRIGHT FIELD | | | |
| FLUORESCENCE | | | |

## FIG. 3

UTR1
UTR2

FIG. 4

EP 4 527 924 A2

# FIG. 5

# FIG. 6

LIGATION

TRANSFORMATION

TREATMENT WITH
RESTRICTION ENZYME

PCR

EP 4 527 924 A2

## FIG. 7

# FIG. 8

FIG. 9

# FIG. 10

# FIG. 11

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2023137652 A
- JP 2024098601 A
- JP 6781854 B

**Non-patent literature cited in the description**

- **DIEZ, M. et al.** iCodon customizes gene expression based on the codon composition. *Sci Rep*, 2022, vol. 12 (1), 12126
- **SAMPLE, P.J. et al.** Human 5' UTR design and variant effect prediction from a massively parallel translation assay. *Nat Biotechnol*, 2019, vol. 37 (7), 803-809
- **EGLOFF, P. et al.** Engineered peptide barcodes for in-depth analyses of binding protein libraries. *Nat Methods*, 2019, vol. 16 (5), 421-428